# EUROPEAN PATENT SPECIFICATION

(11) **EP 3 643 775 B1**
(45) Date of publication and mention of the grant of the patent: **03.07.2024**
(21) Application number: 18820445.7
(22) Date of filing: 22.05.2018
(51) Int. Cl.: C12N 1/20, C05F 11/08, C12R 1/07, A01N 63/22

(54) **BACILLUS AMYLOLIQUEFACIENS QV15 STIMULANT FOR THE SECONDARY METABOLISM OF PHENOLIC COMPOUNDS AND THE INHIBITORY CAPACITY OF RASPBERRY AND STRAWBERRY EXTRACTS FOR ENZYMES RELATED TO METABOLIC SYNDROME**
BACILLUS AMYLOLIQUEFACIENS QV15-STIMULANS FÜR DEN SEKUNDÄRMETABOLISMUS VON PHENOLISCHEN VERBINDUNGEN UND DIE INHIBIERENDE KAPAZITÄT VON HIMBEER- UND ERDBEEREXTRAKTEN FÜR ENZYME IM ZUSAMMENHANG MIT DEM STOFFWECHSELSYNDROM
BACILLUS AMYLOLIQUEFACIENS QV15 STIMULANT DU MÉTABOLISME SECONDAIRE DE COMPOSÉS PHÉNOLIQUES ET DE LA CAPACITÉ INHIBITRICE DES EXTRAITS DE FRAMBOISE ET DE FRAISE SUR LES ENZYMES ASSOCIÉS AU SYNDROME MÉTABOLIQUE

(30) Priority: 21.06.2017 ES 201730818
(43) Date of publication of application: 29.04.2020
(73) Proprietor: Fundación Universitaria San Pablo, 28040 Madrid (ES)
(72) Inventor: GUTIERREZ ALBANCHEZ, Enrique, 28040 Madrid (ES); GUTIERREZ MAÑERO, Francisco Javier, 28040 Madrid (ES); LUCAS GARCIA, Jose Antonio, 28040 Madrid (ES); RAMOS SOLANO, Beatriz, 28040 Madrid (ES)
(86) International application number: PCT/ES2018/070369
(87) International publication number: WO 2018/234599

(56) References cited:
- CN-A- 102 876 603
- US-A1- 2016 183 537
- ABDELHI DIHAZI ET AL: "Use of two bacteria for biological control of bayoud disease caused byin date palm (L) seedlings", PLANT PHYSIOLOGY AND BIOCHEMISTRY, GAUTHIER-VILLARS, PARIS, FR, vol. 55, 2 March 2012 (2012-03-02), pages 7-15, XP028486125, ISSN: 0981-9428, DOI: 10.1016/J.PLAPHY.2012.03.003 [retrieved on 2012-03-14]
- MYUNG-JI SEO ET AL: "Isolation of the Putative Biosynthetic Gene Cluster of 1-Deoxynojirimycin by <i>Bacillus amyloliquefaciens</i> 140N, Its Production and Application to the Fermentation of Soybean Paste", BIOSCIENCE BIOTECHNOLOGY BIOCHEMISTRY, vol. 77, no. 2, 22 May 2014 (2014-05-22), pages 398-401, XP055652175, JP ISSN: 0916-8451, DOI: 10.1271/bbb.120753
- ABDELHI DIHAZI ET AL: "Use of two bacteria for biological control of bayoud disease caused byin date palm (L) seedlings", PLANT PHYSIOLOGY AND BIOCHEMISTRY, vol. 55, 2 March 2012 (2012-03-02), pages 7-15, XP028486125, ISSN: 0981-9428, DOI: 10.1016/j.plaphy.2012.03.003
- MARA LAURA TONELLI ET AL: "Peanut priming induced by biocontrol agents.", PHYSIOLOGICAL AND MOLECULAR PLANT PATHOLOGY, vol. 75, 4 November 2010 (2010-11-04), pages 100-105, XP028131867, ISSN: 0885-5765, DOI: 10.1016/j.pmpp.2010.11.001
- Myung-Ji SEO, Young-Do NAM, So-Young LEE, So-Lim PARK, Sung-Hun YI & Seong-Il LIM: "Isolation of the Putative Biosynthetic Gene Cluster of 1-Deoxynojirimycin by Bacillus amyloliquefaciens 140N, Its Production and Application to the Fermentation of Soybean Paste", Bioscience, Biotechnology, and Biochemistry, vol. 77, no. 2, 22 May 2014 (2014-05-22), pages 398-401, XP055652175, ISSN: 0916-8451, DOI: 10.1271/bbb.120753
- MCDOUGALL GORDON J ET AL: "Different polyphenolic components of soft fruits inhibit alpha-amylase and alpha-glucosidase", Journal of Agricultural and Food Chemistry, vol. 53, 1 April 2005 (2005-04-01), pages 2760-2766, XP002438347, ISSN: 0021-8561, DOI: 10.1021/jf0489926
- HONG Y ET AL.: "Potentiality of Bacillus amyloliquefaciens KFCC11574P isolated from Korean traditional doenjang as a starter in the production of functional soya bean paste.", International Journal of Food Science and Technology, vol. 51, no. 1, 3 November 2015 (2015-11-03), pages 105-113, XP055652189, ISSN: 0950-5423, DOI: 10.1111/ijfs.12973

## Description

This invention relates to a *Bacillus amyloliquefaciens* (QV15, laboratory internal code) strain for application in plant with the aim of improving the synthesis of phenolic compounds of the secondary metabolism with agricultural, pharmacological or nutritional interest, namely, for improving the coloring in strawberry and raspberry fruits, apart from improving the properties of raspberry and strawberry extracts for the inhibitory effect on alpha-glucosidase, ACE and COX2 for improving symptoms or preventing the metabolic syndrome.

This strain, when isolated, was assigned the internal reference of L81, has been deposited for patent purposes in the Spanish Type Culture Collection (CECT), on 31 May 2017, where the number 9371 has been assigned to it. The CECT is located in the Research Building of the University of Valencia, in Burjassot Campus (DP 46100 - Valencia, Spain).

This bacterial strain may serve as a basis for the preparation of different types of stimulant products of the secondary metabolism of plants of agricultural, pharmacological and food interest, and to obtain a greater quantity of active substances and/or novel foods with a standardized phenol content, such as wild berries (strawberries and raspberries) with an increased content in phenolic compounds, in particular anthocyanins, improving coloring and °Brix. These products shall enhance the phenolic bioactive content that may constitute active substances for various medicines, and improve the quality of certain foods. Furthermore, this can be used to improve the properties of blackberry, raspberry and strawberry fruit extracts for the inhibitory effect on alpha-glucosidase and COX2 for improving the metabolic syndrome.

### TECHNICAL FIELD. -

The invention is within the biotechnology, pharmacology and novel food field.

### STATE OF THE ART.-

The mechanisms of action of plant growth promoter bacteria can be summarized in two types: direct, when the produced metabolites impair the plant metabolism (hormonal activity, defensive mechanisms stimulation...) and indirect, when they synthesize the compounds that facilitate the uptake or mobilization of nutrients, or they prevent the growth of pathogenic microorganims without involving the plant, without impairing the plant metabolism.

In this case, it is relevant one of the direct mechanisms, that is, those which impair the plant metabolism. The plant has a secondary metabolism, highly inducible, related to the plant defense and adaptations to adverse situations, to which it has to confront. Within this secondary metabolism we can find the phenolic compound metabolism that, apart from being related to the plant defense, it is of concern for human health, both when foods of plant origin are consumed in which they are naturally contained, and plant extracts for nutritional supplements. They are also important as a source of active substances to obtain medicines. The source of carbon skeletons for nourishing the secondary metabolism is photosynthesis, and any mechanism that may affect this process, will affect the plant health.

*Bacillus amyloliquefaciens* belongs to the group of Gram + bacteria. The genus *Bacillus* is common on soil bacteria, and they can be opportunistic pathogens on animals and plant pathogens. By following the Manual Bergeys taxonomy, March 2001 edition, this bacterium falls within the Bacteria Domain, Phylum Firmicutes, Bacilli Class, Bacillales Order, Bacillaceae Family, Genus *Bacillus, Bamyloliquefaciens* species. The genus *Bacillus* is very common in the edaphic system, and has been repeatedly described as a protective bacterium against different plant diseases. They can produce non-fluorescent catechol-type siderophores that, among other functions, they act as molecules able to capture the iron in the media for the microorganism metabolism. The presence of the *Bacillus* sp. in the rhizosphere of different plants affects beneficially their physiology, meaning that its selection at a rhizosphere level is very likely to occur.

There are many references in the scientific literature that refer to genus *Bacillus* strains as being capable to perform numerous activities of interest in the biotechnology, agriculture and phytopathology field. In the biotechnology field, there are studies i) about their role as biomarkers for sterilization, in biodefence studies, ii) about their influence on the plant primary metabolism by increasing their growth and production, iii) as disinfection agents, iv) as antimicrobial agents for their capability to produce polyketide or lipopeptide type antimicrobial molecules. In the agriculture and phytopathology field there are many references about the capability of *Bacillus amyloliquefaciens* to induce the plant defenses of the plant; on the other hand, there are strains capable to produce chitinases and glucanases, directly protecting against *Alternaria* y *Fusarium* fungi. There are also reference about genus *Bacillus* strains capable to protect against saline stress and against the foliar pathogen *Pseudomonas syringae* DC3000 (Barriuso et al, 2008 Phytopathology), but none about *Bacillus amyloliquefaciens.* Finally, there is no *Bacillus* strain, or *Bacillus amyloliquefaciens* to modulate the biosynthesis pathway of phenylpropanoids, flavonoids and anthocyanins, increasing the concentration of polyphenols, specifically flavonols and anthocyanins.

On the other hand, it is widely known that flavonoid-rich plant extracts, anthocyanins and other phenolic compounds, have a high antioxidant power beneficial to health. Extracts from blueberry species have been frequently cited as healthy products for their antioxidant capacity, neurodegenerative prevention, loss of bone mass prevention, coronary prevention and anticancer effects. Berry extracts, both wild berries and commercial species, have also been linked to hypoglycemic activity, inhibition of adipogenesis, improvement of risk factors for cardiovascular diseases, antiinflammatory capacity and ability to induce satiety and counteract overweight. A recent article of Lila, M.A. (Functional Foods in Health and Disease, 2011, 2:13-24 Page 13 of 24), discusses the impact of bioflavonoids from berries in biomarkers of the metabolic syndrome, associated to diabetes, overweight or obesity conditions and cardiovascular diseases.

Within this field of plant extracts with health benefits, other two recent articles of renowned researchers, Sharma, Kumar (Journal of Diabetology, June 2011; 2:4) y Kaume, Howard, Devareddy (J.10 Agric. Food Chem. 2012, 60, 5716-5727), accurately reflect the state of the art closer to the subject matter of the present invention, in the sense that the blackberry fruits (*Rubus* sp.var Loch Ness), because of their high levels of the referred phenolic compounds, have been related, among other functions, to the glucose-lowering activity, and the ability to induce satiety and counteract overweight. In fact, the Sharma and Kumar article, addresses the antidiabetic effect of *Rubus ellipticus* fruit extracts on diabetes-induced mice through aloxane, although it is worth noting the lack of studies in healthy mice and normal animals.

Hence, following this line of research, the inventive team has managed to prepare methanolic extracts from strawberries and raspberries obtained from plants treated with *Bacillus amyloliquefaciens* QV15 throughout the biological production cycle. These extracts are perfectly characterized in their composition and antioxidant power and have shown to have a greater capacity to inhibit the alpha-glucosidase, ACE and COX2 enzymes, which makes them potentially useful in the preparation of food preparations and drugs for the prevention and improvement of the symptoms associated with metabolic syndrome.

Carrying out a retrospective search of patents worldwide in the Spanish production database Invenet (SPTO) and in the international Worlwide, through the Esp@cenet system, the conclusion drawn from the scientific literature consultation is confirmed: although there is a reference similar to a strain of *Pseudomonas fluorescens* N21.4 (ES 2 336 758 B1), the absence of patent documents related to the bacterial species *Bacillus amyloliquefaciens* that support the ability to stimulate secondary metabolism, specifically the phenylpropanoid, flavonoid and anthocyanin pathway. In this way it is able to modify the metabolic profile of the plant and, therefore, of the health benefits that the fruit or extracts prepared from this plant material may have.

After the aforementioned research, it has been found that there are patents related to the *Bacillus amyloliquefaciens* strain published worldwide, of which none is related to the ability to modulate the metabolic profile of the fruit at a phenolic compound level, in the flavonoid and anthocyanins pathway, this global effect being the subject matter of the present invention. Most patents refer to culture protection through bacterial production of enzymes (glucanases, chitinases, etc.), which exert their protective effect when released outside the cell, at edaphic or foliar level (indirect mechanism), and do not involve the metabolism of the plant in this protection (direct mechanism).

### THE INVENTION. -

The subject matter of the herein described invention and, in view of the previous state of the art, it is understood that complies with the novelty and inventive step conditions required for patenting, is the isolation and characterization of bacterial strain *Bacillus amyloliquefaciens* QV15 (CECT 9371), which is a microorganism from the group of Gram + bacteria, genus *Bacillus,* able to modulate the secondary metabolism, and enhance the properties of the plant extracts for enzymes related to metabolic syndrome (COX2, ACE and alpha-glucosidase).

The physiological characteristics and the genetic analysis of this strain allow us to identify it unequivocally, differentiating it from other species of the genus *Bacillus.*

Once isolated and characterized, with internal reference code L81, various tests were carried out to reveal the potential of this bacterium. These were, production of auxins, degradation of 1-aminocyclopropane-1-carboxylate, phosphate solubilization and production of siderophores and chitinases, proving positive for the production of siderophores.

So far, consistent experiments have been carried out in the inoculation of bacterial suspensions of *Bacillus amyloliquefaciens* QV15 on *Arabidopsis thaliana,* improving photosynthesis (cpPSII/NPQ), without affecting plant growth. In these experiments, an increase in SOD activity and a decrease in APX activity and the rest of *ROS scavenging* activities has been detected, together with a moderate increase in the glucanase (PR2) and chitinase (PR3) activities of the plant. When these plants previously inoculated with QV15 are subjected to shock with *Xanthomonas campestris* pv tomato, the plants withstood this attack much better, presenting a protection of 60%, associated with a more than a twofold increase that in the controls in PR2 and PR3 activity.

On the other hand, field experiments have been carried out on blackberry *(Rubus* var Loch Ness), raspberry *(Rubus idaeus)* and strawberry *(Fragaria vesca)* plants in production greenhouses. Applications have been made with QV15 at the root level, from September to February, every 15 days, under counter-season production conditions. An increase in the synthesis of phenolic compounds is detected in all of them; in blackberry, this increase is controlled at a level of transcription factors, stimulating the MYB6 transcription, and certain genes of the flavonol biosynthesis pathway.

These experiments carried out on different plant species show that *Bacillus amyloliquefaciens* QV15 is able to modulate the secondary metabolism of plants, as well as to improve the properties of raspberry and strawberry fruit extracts, improving the inhibitory effect of alpha glucosidase, ACE and COX2 with respect to non-inoculated controls and, therefore, this bacterium can be used in any type of culture of agronomic, pharmacological or nutritional, agricultural or forestry interest, for the improvement of symptoms or prevention of metabolic syndrome.

*Bacillus amyloliquefaciens* QV15 can be applied to blackberry *(Rubus var Loch* Ness), raspberry *(Rubus idaeus)* and strawberry (*Fragaria vesca*) plants by modifying the content of phenolic compounds in leaves and fruits, especially in flavonols, anthocyanins and catechins derivatives. In blackberry leaves it acts at a transcription factor level, as well as in certain genes of the biosynthesis pathway of flavonols and anthocyanins, and thereby modifies the metabolic profile (flavonols and derivatives) in leaves. In blackberry fruits, it also acts at a transcription factor level and in certain genes of the biosynthesis pathway of flavonols and anthocyanins. This can be applied to any plant species from which red berries or wild fruits form part, such as strawberry, raspberry, blackberry, blueberry, or bilberry, or grape, in order to increase their content in phenolic compounds of pharmacological, nutritional interest, specifically in raspberry and strawberry to improve the anthocyanin content and, therefore, its coloration.

The aforementioned experiments on the use of *Bacillus amyloliquefaciens* QV15 as a stimulant of the secondary metabolism of phenolic compounds, specifically anthocyanins in strawberry and raspberry, and to improve extracts on the action of the alpha glucosidase, ACE and COX2 enzymes are presented at the end of this specification, within the section of embodiment.

The purpose that is ultimately pursued with this invention and that constitutes the technical advantage provided with it, is to have a bacteria that stimulates the secondary metabolism of phenolic compounds in plants of agronomic, pharmacological, nutritional interest achieving a double effect: on the one hand, to improve the anthocyanin content in strawberries and raspberries, and therefore, their commercial value, and on the other hand, to obtain the plant raw material source of improved extracts with respect to non-inoculated controls for their effect on alpha-glucosidase, ACE and COX2 enzymes and, therefore, they can be used for the improvement of symptoms or prevention of metabolic syndrome.

Accordingly, with the present patent application the use of the *Bacillus amyloliquefaciens* QV15 strain, or any fraction thereof, is claimed for its application in any type of plant species, forming part of any preparation, either individually or in combination with other organisms, in order to stimulate the secondary metabolism of phenolic compounds in plants of agronomic, pharmacological, nutritional interest and, at the same time, it improves the extracts obtained from plant material treated with the strain, on the action of alpha-glucosidase, ACE and COX2.

### EMBODIMENT. -

Studying the rhizosphere of two species of the genus *Pinus,* selected for their forest interest, we find the genus *Bacillus* strain that is here reviewed.

The strain was isolated from the rhizosphere of a natural population of *Pinus pinea L.* During the isolation of bacteria carried out in the rhizosphere of two pine species (*Pinus pinaster Aiton* and *Pinus pinea L*.) and in the mycosphere of the mycorrhizal fungus associated to both, *Lactarius deliciosus* (Fries) S.F. Gray., in autumn of 2000, coinciding with the fruiting period of *Lactarius deliciosus,* in the Sierra de Aracena (Huelva, Spain). As a result of this sampling, 720 strains were collected, including *Bacillus amyloliquefaciens* (QV15, internal laboratory code). The isolation of said strain was performed on nutrient agar (PCA).

In the laboratory, this microorganism is maintained with a high survival rate in 20% glycerol in nutrient broth (Pronadisa) at -80 °C or in 15% glycerol in water at -20 °C and is easily recovered in the culture media used for insulation in both solid phase and liquid phase at 28 °C.

For the strain characterization different phenotypic characters were considered, which are detailed in this specification: (i) colony morphology (ii) cell morphology, (iii) sequencing of the ribosomal DNA gene corresponding to the 16S subunit. iv) Genome sequencing
Morphological, biochemical and genetic characteristics of *Bacillus amyloliquefaciens QV15.-*

Taxonomic characterization of *Bacillus amyloliquefaciens* was performed by identifying the strain by 16S ribosomal DNA partial sequencing, its comparison with the sequences in the databases revealed a 100% homology with a *Bacillus amyloliquefaciens* strain.

Next, the colony morphology is specified at 24 h of incubation at 28° on standard methods agar (PCA).

**TABLE 1**

| | QV15 |
|---|---|
| Colony size | < 1 mm∅ |
| Shape | circular |
| Edge | Smooth |
| Transparency | No |
| Consistency | Creamy |
| Color | Dark yellow |

Growing in liquid media (Lennox Pronadisa) the color of the media changes to darker yellow from the exponential phase of growth to the stationary phase of growth.

The morphological characters of *Bacillus amyloliquefaciens QV15* at 24 h of incubation at 28° on standard methods agar (PCA) correspond to a sporulated Gram positive bacillus.

Then, the strain genetic analysis was carried out for identification, for this, the following steps were followed:

### DNA extraction. -

For DNA extraction, colonies grew for 24 hours in Lennox (Pronadisa) at 28 °C under agitation. After this time, the genomic DNA of each bacterium was extracted with the Ultraclean ^{™} Microbial DNA isolation kit (MoBio, CA, USA), according to the manufacturer's instructions.

### 16s rDNA Amplification. -

The 1500 bp corresponding to this region were amplified by PCR with the following primers: forward 5'-AGA GTT TGA TCC TGG CTC AG-3' and reverse 5'-AAG GAG GTG ATC CAG CCG CA-3' (Ulrike, 1989), in a reaction of 25 µL with 1X of 10X buffer, 2.5 µM MgCl₂, 250 µM of each DNTP, 2.5 µM forward primer, 2.5 µM reverse primer, 1.25 units of DNA polymerase (AmpliTaq Applied) and 100 ng of bacterial DNA. The amplification was performed in a GeneAmp 2700 thermal cycler (Applied Biosystems) with the following conditions: 95 °C 5 minutes, followed by 25 cycles of 94 °C 30 seconds, 65.5 °C 30 seconds and 72 °C 30 seconds, ending with 7 minutes at 72 °C.

### Gel Visualization. -

The PCR product was re-solved on 1% agarose gel (w/v) in Tris-Acetate-EDTA buffer (1% TAE) with ethidium bromide (0.5 mg/mL) and visualized on an image analyzer GelDoc2000TM 170-8126 (Biorad, CA, USA).

### DNA sequencing. -

Once the amplification was verified, the PCR product was purified with the UltraClean ^{™} PCR Clean-up DNA purification kit (MoBio, CA, USA), it was sequenced UNlOAD DE GENÓMICA PARQUE CIENTIFICO DE MADRID-U.C.M. in a ABI PRIMS^{®} 377 ADN Sequencer (Applied Biosystems, CA, USA).

### Sequence Computer analysis. -

The sequences were aligned with the Bioedit Sequence Aligment editor 5.0.3 program. ^{®}, were manually reviewed and corrected and analyzed by BLASTN 2.2.6 (Altschul et al., 1997) in the GeneBank EMBL and DDBJ (NCBI BLASTR website: http://www.ncbi.nlm.nih.gov/), resulting in the highest homology: *Bacillus amyloliquefaciens QV15* 16S ribosomal RNA gene, complete sequence, and the sequence being deposited in the GeneBank with accession number AY307364.1

### Bacillus amyloliquefaciens as an enhancer of adaptation to stress conditions and a stimulant of the primary and secondary metabolism of phenolic compounds. -

1^{st.} Elicitation experiment in *Arabidopsis thaliana* seedlings by inoculating the bacteria twice at the root level, in the fourth and fifth weeks after the germination of the seeds. Three days after the second inoculation, the pathogen *(Xantomonas campestris)* is inoculated at a foliar level by pathogen spraying. Photosynthesis was determined by fluorescence (Fo, Fv/Fm, ΦPSII, and NPQ), enzymatic activities (ROS scavenging cycle, and defense enzymes), and the relative disease index. It was observed: i) an increase in SOD activity and a decrease in APX, ii) an increase in the activity of glucanases, chitinases, and cellulases, in the treatment with QV15+pathogen, while in QV15 they maintain a similar or lower activity than the control, demonstrating an induction of systemic defense, iii) a reduction in the disease symptomatology caused by the attack of the pathogen of 63.61%.
2^{nd}. Elicitation experiment in *Rubus var Loch Ness* plants by applying the bacteria at the root. - A bacterial suspension of QV15 strain was inoculated into the root of *Rubus var Loch Ness* plants every two weeks (September 2014 to February 2015), since the transplant. Photosynthesis was determined by fluorescence (Fo, Fv/Fm, ΦPSII, and NPQ), enzymatic activities (ROS cycle, and defense enzymes), leaf bioactives (total phenols, flavonols and anthocyanins), chlorophylls, in two moments of sampling (flowering and maximum fruiting); at maximum fruiting, the nutritional parameters (pH, °Brix, and citric acid%) and bioactive compounds in fruits were determined; the gene expression of the flavonoid and defense protein pathway in leaves and fruits collected in fruiting has been studied; and finally, measurements have been made with fruit extracts on the inhibition for enzymes related to glucose regulation (alpha-amylase and alpha-glucosidase), on hypertension (ACE), and on inflammation (COX2), enzymes related to metabolic syndrome. It was observed: i) at a photosynthesis level a ΦPSII increases and NPQ decreases ii) induction of SOD activity, decrease in APX, iii) increase in activity and expression of defense proteins (glucanases, and chitinases), both in flowering and in fruition, related to an increase in defense against pathogens, specifically against Mildew, iv) at a bioactive components in leaves level, an increase in phenols is observed in flowering, although flavonols and anthocyanins are not impaired, while in fruiting they all decrease in leaves, v) increase in chlorophyll A, B and total in both flowering and fruiting, vi) in fruits a decrease in phenols was observed, while flavonols and anthocyanins were not impaired, indicating modification of secondary metabolism vii ) in blackberry fruits, an increase in antioxidant potential, viii) increase in gene expression of the flavonol and anthocyanin pathway CHS, F3H, DFR, LAR, and GST1 (in fruit and leaves), data that coincide with the bioactive components analysis, and that suggest a deviation from the flavonol pathway towards the production of catechins in intermediate stages of maturation.

The extracts were prepared from fresh blackberries and leaves of the "Loch Ness" variety. For the extracts used to measure the effect on alpha-glucosidase, ACE, and COX2, the blackberries were first lyophilized, then extracted with 80% methanol, centrifuged and the organic fraction vacuum evaporated. The extract with 20% water was characterized; while for the extracts used for the rest of the measurements, an extraction with 80% methanol was performed. So, the measurements obtained for alpha-glucosidase, ACE, and COX2 would be in dry weight while for the rest in fresh weight.

For the characterization of the extract the following determinations were made:
The total phenolic content of the extract is determined by the colorimetric method of Singleton V.L., Rossi J.A. (1965) Colorimetry of total phenolics with phosphomolibdicphosphotungstic acid reagent. Am J Enol Vitic 10 16,144-158, which is based on the oxidation in basic medium of the hydroxyl groups of the phenols by the Folin-Ciocalteu reagent. The results are expressed as mg of gallic acid/g of extract. In this way, the extracts obtained following the processes detailed below have a minimum total phenolic content of 20 mg/g.

The total flavonol content of the extract is determined by the colorimetric method of aluminum chloride from Zishen et al. (1999) Zhishen J, Mengcheng T, Jianming W (1999) The determination of flavonoid contents in mulberry and their scavenging effects on superoxide radicals. Food Chem 64: 555-559. Doi: 20 10.1016 / S0308-8146 (98) 00102-2 with modifications. It is expressed as equivalent mg of catechin per gram of fresh extract.

The total anthocyanin content was determined by the differential pH method described by Giusti and Wrolstad (2001), Giusti MM, Wrolstad RE (2001) Anthocyanins Characterization and measurement with UV-visible spectroscopy. In: Wrolstad RE, Acree TE, An H, Decker EA, Penner MH, Reid DS, Schwartz SJ, Shoemaker CF, Sporns P, Wiley J (ed) Current Protocols in Food Analytical Chemistry. New York, pp F121-F129. doi: 10.1002 / 0471142913.faf0102s00, which evaluates the different absorbance of anthocyanins at different pH. The results are expressed as mg 30 of cyanidine-3-glycoside per gram of fresh extract.

For the measurement of enzymatic activities related to defense and oxidative stress, the methods described in Garci-Limones, C., Hervás, A., Navas-Cortés, JA, Jiménez-Díz, RM, & Tena, M. (2002) were used). Induction of an antioxidant enzyme system and other oxidative stress markers associated with compatible and incompatible interactions between chickpea ( Cicer arietinum L.) and Fusarium oxysporum f. sp. ciceris. Physiological and Molecular Plant Pathology, 61(6), 325-337. Lee, B.-R., Jung, W. J., Lee, B.-H., Avice, J.-C., Ourry, A., & Kim, T. H. (2008). Kinetics of drought-induced pathogenesis-related proteins and its physiological significance in white clover leaves. Physiologia Plantarum, 132(3), 329-337. Saravanakumar, D., Lavanya, N., Muthumeena, B., Raguchander, T., Suresh, S., & Samiyappan, R. (2008). Pseudomonas fluorescens enhances resistance and natural enemy population in rice plants against leaffolder pest. Journal of Applied Entomology, 132(6), 469-479. Xu, C., Natarajan, S., & Sullivan, J. H. (2008). Impact of solar ultraviolet-B radiation on the antioxidant defense system in soybean lines differing in flavonoid contents. Environmental and Experimental Botany, 63(1-3), 39-48.

For the chlorophyll measurements, the method described in Harmut K. Lichtenthaler and Claus Buschmann (2001) was followed. Extraction of Photosynthetic Tissues: Chlorophylls and Carotenoids. Current Protocols in Food Analytical Chemistry F.4.2.1- F.4.2.1.

3^{rd}. Elicitation experiment in raspberry plants *(Rubus idaeus var Adelita),* by inoculating the bacteria at a root level, every two weeks, from October 2015 to May 2016, analyzing the two production maximums of raspberries (January and May). Photosynthesis was determined by fluorescence(Fo, Fv/Fm, ΦPSII, y NPQ), bioactive compounds (phenols, flavonones and anthocyanins) in leaves and fruits, nutritional values in fruits (pH, °Brix, and citric acid%); and finally, measurements have been made with the methanolic fruit extracts on the inhibition of enzymes related to glucose regulation (alpha amylase and alpha glucosidase), hypertension (ACE) and on inflammation (COX2), enzymes related to the metabolic syndrome. It was observed in fruits: i) an increase in °Brix, reduction in the amount of citric acid, as well as a decrease in pH, ii) increase in the amount of anthocyanins, phenols and flavonols, with respect to control, iii) increase in the ability of fruit extracts to inhibit alpha glucosidase, ACE and COX2. The content of anthocyanins, phenols and flavonols in fruits of inoculated and control plants appears in table 2; the IC50 of α glucosidase, and the inhibition% of ACE and COX2 appear in table 3.

**Table 2.**

| | Phenols (mg Gallic Equivalents /100 g fresh weight) | | Flavonols (mg (+)-catechin(195) Equivalents /100g fresh weight) | | Anthocyanins (mg cyanidine-3-glycoside Equivalents /100g fresh weight) | |
|---|---|---|---|---|---|---|
| | Winter | Spring | Winter | Spring | Winter | Spring |
| Control | 154.17± 7.7 | 389.47± 10.0 | 6.06± 0.24 | 87.46± 1.18 | 9.39± 1.27 | 23.52± 1.5 |
| QV15 | 248.45± 6.4 | 379.44± 7.45 | 11.28± 0.92 | 77.67± 1.18 | 4.62± 0.19 | 21.19± 1.76 |

**Table 3.**

| | α Glucosidase (IC50, mg of dry extract/ml) | | ACE inhibition %[10 mg of dry extract/ml] | | COX2 inhibition % [10 mg of dry extract/ml] | |
|---|---|---|---|---|---|---|
| | Winter | Spring | Winter | Spring | Winter | Spring |
| Control | 3.80± 0.16 | 4.66± 0.05 | 92.15± 0.04 | 91.180.02 | 29.59± 0.87 | 29.02± 0.25 |
| QV15 | 3.32± 0.04 | 3.36± 0.22 | 91.88± 0.01 | 92.09± 0.07 | 32.24± 0.88 | 32.40± 0.49 |

The raspberry extract was prepared from fresh raspberries of the "Adelita" variety. For the extracts used to measure the effect on alpha-glucosidase, ACE, and COX2, the blackberries were first lyophilized, then extracted with 80% methanol, centrifuged and the organic fraction vacuum evaporated. The extract with 20% water was characterized; while for the extracts used for the rest of the measurements, an extraction with 80% methanol was performed. So, the measurements obtained for alpha-glucosidase, ACE, and COX2 would be in dry weight while for the rest in fresh weight.

For the characterization of the extract the following determinations were made:
The total phenolic content of the extract is determined by the colorimetric method of Singleton V.L., Rossi J.A. (1965) Colorimetry of total phenolics with phosphomolibdicphosphotungstic acid reagent. Am J Enol Vitic 10 16,144-158, which is based on the oxidation in basic medium of the hydroxyl groups of the phenols by the Folin-Ciocalteu reagent. The results are expressed as mg of gallic acid/g of extract. In this way, the extracts obtained following the processes detailed below have a minimum total phenolic content of 20 mg/g.

The total flavonol content of the extract is determined by the colorimetric method of aluminum chloride from Zishen et al. (1999) Zhishen J, Mengcheng T, Jianming W (1999) The determination of flavonoid contents in mulberry and their scavenging effects on superoxide radicals. Food Chem 64: 555-559. Doi: 20 10.1016 / S0308-8146 (98) 00102-2 with modifications. It is expressed as equivalent mg of catechin per gram of fresh extract.

The total anthocyanin content was determined by the differential pH method described by Giusti and Wrolstad (2001), Giusti MM, Wrolstad RE (2001) Anthocyanins Characterization and measurement with UV-visible spectroscopy. In: Wrolstad RE, Acree TE, An H, Decker EA, Penner MH, Reid DS, Schwartz SJ, Shoemaker CF, Sporns P, Wiley J (ed) Current Protocols in Food Analytical Chemistry. New York, pp F121-F129. doi: 10.1002 / 0471142913.faf0102s00, which evaluates the different absorbance of anthocyanins at different pH. The results are expressed as mg 30 of cyanidine-3-glycoside per gram of fresh extract.

4^{th}. Elicitation experiment in strawberry plants *(Fragaria vesca var Fortuna)* by inoculating after transplantation every two weeks at a root level throughout the plant cycle (January to May 2016). Photosynthesis was determined by fluorescence(Fo, Fv/Fm, ΦPSII, y NPQ), in the middle of the production cycle (March 2016) and at the end (May 2016), the bioactive compounds (phenols, flavonols and anthocyanins) and nutritional values (pH, °Brix, and citric acid%) in fruits were determined at both times;; and finally, measurements have been made with the methanolic fruit extracts on the inhibition of enzymes related to glucose regulation (alpha amylase and alpha glucosidase), hypertension (ACE) and on inflammation (COX2), enzymes related to the metabolic syndrome. It was observed: i) at a photosynthesis level, a decrease in Fo and NPQ, so it is understood that the plant is less stressed than the control and loses less energy from photosynthesis in the form of heat, so it will be used to the generation of primary or secondary metabolites ii) an increase in anthocyanins, iii) an increase in °Brix, iv) a decrease in rotten fruit and an increase in premium quality fruit, v) an increase in the properties of the fruits extracts on alpha glucosidase, ACE and COX2. The content of anthocyanins, phenols and flavonols in fruits of inoculated and control plants appears in table 4; the IC50 of α glucosidase, and the inhibition% of ACE and COX2 appear in table 5.

**Table 4.**

| | Phenols (mg Gallic Equivalents /100 g fresh weight) | | Flavonols (mg (+)-catechin(195) Equivalents /100g fresh weight) | | Anthocyanins (mg cyanidine-3-glycoside Equivalents /100g fresh weight) | |
|---|---|---|---|---|---|---|
| | Winter | Spring | Winter | Spring | Winter | Spring |
| Control | 315.79± 6.86 | 188.24± 2.74 | 80.15± 0.37 | 38.40± 0.49 | 17.24± 1.62 | 21.19± 5.61 |
| QV15 | 315.79± 22.41 | 202.50± 2.75 | 59.38± 1.35 | 29.30± 0.55 | 39.11± 0.19 | 20.28± 2.6 |

**Table 5.**

| | α Glucosidase (IC50, mg of dry extract/ml) | | ACE inhibition %[10 mg of dry extract/ml] | | COX2 inhibition % [10 mg of dry extract/ml] | |
|---|---|---|---|---|---|---|
| | Winter | Spring | Winter | Spring | Winter | Spring |
| Control | 11.56± 1.05 | 8.27± 0.43 | 95.2± 0.06 | 99.06± 0.02 | 35.39± 0.21 | 33.76± 0.32 |
| QV15 | 9.99± 0.1 | 10.04± 0.56 | 92.41± 0.01 | 91.98± 0.14 | 36.61± 1.15 | 34.12± 0.55 |

The strawberry extract was prepared from fresh strawberries of the "Fortuna" variety. For the extracts used to measure the effect on alpha-glucosidase, ACE, and COX2, the blackberries were first lyophilized, then extracted with 80% methanol, centrifuged and the organic fraction vacuum evaporated. The extract with 20% water was characterized; while for the extracts used for the rest of the measurements, an extraction with 80% methanol was performed. So, the measurements obtained for alpha-glucosidase, ACE, and COX2 would be in dry weight while for the rest in fresh weight.

5^{th}. Elicitation experiment in strawberry plants *(Fragaria vesca var Fortuna)* by inoculating after transplantation every two weeks at a root level throughout the plant cycle (October to March 2017). Photosynthesis was determined by fluorescence (Fo, Fv/Fm, ΦPSII, and NPQ) in the middle of the production cycle (March 2017), and at the end of the cycle (March 2017), bioactive compounds (phenols, flavonols, and anthocyanins) and nutritional values(pH, °Brix, and citric acid%) and the size of the fruit were determined. It was observed: i) an increase in the amount of larger fruit after inoculations ii) an increase in anthocyanins, iii) an increase in flavonols, iv) a decrease in citric acid%.

The strawberry extract was prepared from fresh strawberries of the "Fortuna" variety. An extraction with 80% methanol was performed.

### INDUSTRIAL APPLICABILITY. -

Given the above-mentioned properties of *Bacillus amyloliquefaciens QV15* as a secondary metabolism stimulator, this bacterial strain has a specific application in the agri-food, chemical and pharmaceutical industry, being able to be used as part of any preparation (individually or in combination with other microorganisms ) and bringing it into contact (to the strain or any part of it) with the seed, the root or aerial system of the plants by any means available, in any plant species, or in any form of *in vitro* culture, to increase the concentration of secondary metabolites of phenolic nature with pharmacological and/or nutritional interest. In raspberry and strawberry, to improve the color in the counter-season production, specifically due to the increase of anthocyanins; and to obtain enhanced extracts by their ability to inhibit alpha glucosidase, ACE and COX2 enzymes.

## Claims

1. ***Bacillus amyloliquefaciens*** QV15 deposited as CECT 9371, a microorganism from the group of Gram + bacteria, genus *Bacillus,* **characterized by** its capacity to stimulate the secondary metabolism of phenolic compounds in plant species, namely, flavonoids, anthocyanins, catechins and its capacity to increase the Brix degrees.

2. *Bacillus amyloliquefaciens* QV15 deposited as CECT 9371 according to claim 1, **characterized by** its capacity to increase the anthocyanin content in strawberry and raspberry fruits.

3. *Bacillus amyloliquefaciens* QV15 deposited as CECT 9371, a microorganism from the group of Gram + bacteria, genus *Bacillus,* **characterized by** its capacity to enhance the properties of the extracts of raspberry and strawberry fruit as inhibitors for enzymes related to metabolic syndrome: alpha-glucosidase, blood glucose regulators, ACE, angiotensin converter for hypertension and COX2, inflammation.

4. Use of *Bacillus amyloliquefaciens* QV15 deposited as CECT 9371 according to claims 1 to 3, for application to any type of culture of agricultural, pharmacological or nutritional interest, in the agriculture or forestry sector, in order to increase bioactives and/or enhance the extracts of leaf and/or fruit for the effect thereof on alpha-glucosidase, ACE and COX2.

5. Use of *Bacillus amyloliquefaciens* QV15 deposited as CECT 9371 according to claims 1 to 3, for application to any plant species belonging to *Rubus* sp. family.

6. Use of *Bacillus amyloliquefaciens* QV15 deposited as CECT 9371 according to claims 1 to 3, for application to any plant species from which red berries or wild fruits form part (such as strawberry, raspberry, blackberry, blueberry or bilberry) or those from grapes, with the aim of improving the organoleptic qualities and the coloring in fruits, particularly, from anthocyanins.

7. Use of *Bacillus amyloliquefaciens* QV15 deposited as CECT 9371 according to claims 1 to 3, either the individual strain or in combination with other organisms, forming part of any preparation, either individually or in combination with other organism, and by any available means in which the bacteria contacts the seed, the root or aerial system of plants.

8. Use of Bacillus amyloliquefaciens QV15 deposited as CECT 9371 according to claims 1 to 3, either the individual strain or in combination with other organisms, or forming part of any preparation, either individually or in combination with other organism, and by any available means in which the bacteria contacts the plant cells in any differentiation state on an *in vitro* culture.

## Patentansprüche

1. ***Bacillus amyloliquefaciens*** QV15, hinterlegt als CECT 9371, ein Mikroorganismus aus der Gruppe der Gram+-Bakterien, Gattung Bacillus, **gekennzeichnet durch** seine Fähigkeit auszeichnet, den Sekundärstoffwechsel phenolischer Verbindungen in Pflanzenarten zu stimulieren, nämlich Flavonoide, Anthocyane, Catechine und seine Fähigkeit, den Brix-Grad zu erhöhen,

2. *Bacillus amyloliquefaciens* QV15, hinterlegt als CECT 9371, nach Patentanspruch 1, **gekennzeichnet durch** seine Fähigkeit, den Anthocyangehalt in Erdbeer- und Himbeerfrüchten zu erhöhen.

3. *Bacillus amyloliquefaciens* QV15, hinterlegt als CECT 9371, ein Mikroorganismus aus der Gruppe der Gram+-Bakterien, Gattung Bacillus, **gekennzeichnet durch** seine Fähigkeit, die Eigenschaften der Extrakte von Himbeer- und Erdbeerfrüchten als Inhibitoren für Enzyme, die mit dem metabolischen Syndrom zusammenhängen, zu verbessern: Alpha-Glucosidase, Blutzuckerregulatoren, ACE, Angiotensin-Konverter für Bluthochdruck und COX2, Entzündung.

4. Verwendung von *Bacillus amyloliquefaciens* QV15, hinterlegt als CECT 9371, gemäß den Patentansprüchen 1 bis 3, zur Anwendung bei jeder Art von Kultur von landwirtschaftlichem, pharmakologischem oder ernährungsphysiologischem Interesse im land- oder forstwirtschaftlichen Sektor, um die Bioaktivität zu erhöhen und/oder die Extrakte von Blättern und/oder Früchten hinsichtlich ihrer Wirkung auf Alpha-Glucosidase, ACE und COX2 zu verbessern.

5. Verwendung von *Bacillus amyloliquefaciens* QV15, hinterlegt als CECT 9371, gemäß den Patentansprüchen 1 bis 3, zur Anwendung bei jeder Pflanzenart, die zur Familie Rubus sp. gehört,

6. Verwendung von *Bacillus amyloliquefaciens* QV15, hinterlegt als CECT 9371, gemäß den Patentansprüchen 1 bis 3, zur Anwendung bei allen Pflanzenarten, zu denen rate Beeren oder Wildfrüchte gehören (wie Erdbeere, Himbeere, Brombeere, Heidelbeere oder Blaubeere), oder bei Weintrauben, mit dem Ziel, die organoleptischen Eigenschaften und die Färbung von Früchten, insbesondere von Anthocyanen, zu verbessern.

7. Verwendung von *Bacillus amyloliquefaciens* QV15, hinterlegt als CECT 9371, gemäß den Patentansprüchen 1 bis 3, entweder als Einzelstamm oder in Kombination mit anderen Organismen, als Teil einer beliebigen Zubereitung, entweder einzeln oder in Kombination mit anderen Organismen, und durch jedes verfügbare Mittel, bei dem das Bakterium mit dem Samen, dem Wurzel- oder Luftsystem von Pflanzen in Kontakt kommt.

8. Verwendung von *Bacillus amyloliquefaciens* QV15, hinterlegt als CECT 9371, nach den Patentansprüchen 1 bis 3, entweder als einzelner Stamm oder in Kombination mit anderen Organismen, oder als Teil einer beliebigen Zubereitung, entweder einzeln oder in Kombination mit einem anderen Organismus, und durch jedes verfügbare Mittel, bei dem das Bakterium die Pflanzenzellen in einem beliebigen Differenzierungszustand auf einer In-vitro-Kultur kontaktiert.

## Revendications

1. ***Bacillus** amyloliquefaciens* **QV15 déposé sous le numéro** CECT 9371, micro-organisme du groupe des bactéries Gram +, du genre Bacillus, **caractérisé par** sa capacité à stimuler le métabolisme secondaire des composés phénoliques des espèces végétales, à savoir les flavonoïdes, les anthocyanes et les catéchines, et par sa capacité à augmenter les degrés Brix.

2. *Bacillus amyloliquefaciens* QV15 déposé sous le numéro CECT 9371, selon la revendication 1, **caractérisé par** sa capacité à augmenter la teneur en anthocyanes des fruits de fraises et de framboises.

3. *Bacillus amyloliquefaciens* QV15 déposé sous le numéro CECT 9371, micro-organisme du groupe des bactéries Gram +, genre Bacillus, **caractérisé par** sa capacité à renforcer les propriétés des extraits de framboises et de fraises en tant qu'inhibiteurs d'enzymes liées au syndrome métabolique : alpha-glucosidase, régulateurs de la glycémie, ACE, convertisseur d'angiotensine pour l'hypertension et COX2, inflammation.

4. Utilisation de *Bacillus amyloliquefaciens* QV15 déposé sous le numéro CECT 9371, selon les revendications 1 à 3, pour application à tout type de culture d'intérêt agricole, pharmacologique ou nutritionnel, dans le secteur agricole ou forestier, afin d'augmenter les bioactifs et/ou d'améliorer les extraits de feuilles et/ou de fruits pour leur effet sur l'alpha-glucosidase, l'ECA et la COX2.

5. Utilisation de *Bacillus amyloliquefaciens* QV15 déposé sous le numéro CECT 9371, selon les revendications 1 à 3, pour application à toute espèce végétale appartenant à la famille des Rubus sp.

6. Utilisation de *Bacillus amyloliquefaciens* QV15 déposé sous CECT 9371, selon les revendications 1 à 3, pour application à toute espèce végétale dont font partie les baies rouges ou fruits sauvages (tels que fraise, framboise, mûre, myrtille ou myrtille) ou ceux issus du raisin, dans le but d'améliorer les qualités organoleptiques et la coloration des fruits, en particulier, à partir des anthocyanes.

7. Utilisation de *Bacillus amyloliquefaciens* QV15 déposé comme CECT 9371, selon les revendications 1 à 3, soit la souche individuelle ou en combinaison avec d'autres organismes, faisant partie de toute préparation, soit individuellement ou en combinaison avec d'autres organismes, et par tout moyen disponible dans lequel la bactérie entre en contact avec la graine, la racine ou le système aérien des plantes.

8. Utilisation de *Bacillus amyloliquefaciens* QV15 déposé sous le numéro CECT 9371, selon les revendications 1 à 3, soit la souche individuelle, soit en combinaison avec d'autres organismes, ou faisant partie de toute préparation, soit individuellement, soit en combinaison avec d'autres organismes, et par tout moyen disponible dans lequel la bactérie entre en contact avec les cellules végétales dans n'importe quel état de différenciation sur une culture in vitro.
